# EUROPEAN PATENT SPECIFICATION

(11) **EP 2 158 897 B1**
(45) Date of publication and mention of the grant of the patent: **04.11.2015**
(21) Application number: 09168279.9
(22) Date of filing: 20.08.2009
(51) Int. Cl.: A61K 8/891, A61Q 1/12, A61K 8/04

(54) **Cosmetic product with smoothing effect and rubbery texture which turns into powder when applied and process for obtaining the same.**
Kosmetikprodukt mit Glättungseffekt und gummiartiger Textur, das sich beim Auftragen in Puder umwandelt sowie Verfahren zum Erhalt desselben
Produit cosmétique ayant un effet lisseur et une texture caoutchouteuse qui se transforme en poudre lorsqu´il est appliqué et son procédé de fabrication

(30) Priority: 27.08.2008 IT MI20081541
(43) Date of publication of application: 03.03.2010
(73) Proprietor: Intercos S.p.A., 20122 Milano (IT)
(72) Inventor: Mandelli, Antonio, 20063, Cernusco sul Naviglio (MI) (IT); Rando, Pietro, 20090, Opera (MI) (IT)
(74) Representative: Mittler, Enrico

(56) References cited:
- US-A1- 2005 112 072
- US-A1- 2007 098 661
- ANONYMOUS: "Gransil OL" INTERNET ARTICLE - GRAND INDUSTRIES, [Online] XP002538325 Retrieved from the Internet: URL:http://www.gransil.cn/en/cosmetics/for mula_bases/gransil_ol.php> [retrieved on 2009-07-01]

## Description

The present invention relates to a cosmetic product with smoothing effect and rubbery texture which turns into powder when applied, as well as to the process for obtaining the same. The aforesaid cosmetic product may be used as a smoothing-effect, shine-free makeup foundation.

A cosmetic product of this kind, which uses polymethylsilsesquioxane in association with silicone oils as basic ingredient, is already commercialized.

This product appears in the form of compact powder which, once applied, conceals pores, absorbs sebum in excess and reduces oiliness in excess without causing weals or leaving white residues on the skin.

Such a product has problems of stability, however, which make it unusable over time because it breaks very easily when applied.

US 2007/098661 A1 discloses powder putties which uses corn starks as cosmetically accepted powder.

US 2005/112072 A1 discloses a silicone gel comprising a hybrid polymethylsisesquioxane (PMSQ)/polydimethylsilozane powder.

It is the object of the present invention to provide a similar product which does not have the abovementioned negative effects.

In accordance with the present invention, such an object is achieved by a cosmetic product with smoothing effect characterized in that it comprises:
- from 60 to 70 % by weight of polymethylsilsesquioxane;
- from 30 to 40 % by weight of silicone oils and resins.

In particular, the polymethylsilsesquioxane used is Tospearl 3000 A PC from Momentive.

The silicone oils and resins may consist of:
- from 20 to 30 % by weight of dimethicone;
- from 5 to 10 % by weight of dimethicone and dimethicone crosspolymer.

Preferably, dimethicone with low viscosity (e.g. from 10 to 350 cst) is used, such as Fluid 200/100 from Dow Corning, and dimethicone and dimethicone crosspolymer used is D.C. 9041 from the same manufacturer.

Active ingredients, solar filters, pigments, pearls and so on may be present.

The following examples are merely provided by way of non-limiting example:

### EXAMPLE 1

| **Formula 1** | **% by weight** |
|---|---|
| Dimethicone 350 cst | 30.00 |
| Dimethicone and dimethicone crosspolymer | 5.00 |
| Polymethylsilsesquioxane | 65.00 |

### EXAMPLE 2

| **Formula 2** | **% by weight** |
|---|---|
| Dimethicone 350 cst | 28.00 |
| Dimethicone and dimethicone crosspolymer | 7.00 |
| Polymethylsilsesquioxane | 65.00 |

### EXAMPLE 3

| **Formula 3** | **% by weight** |
|---|---|
| Dimethicone 100 cst | 26.00 |
| Dimethicone and dimethicone crosspolymer | 9.00 |
| Polymethylsilsesquioxane | 65.00 |

### EXAMPLE 4

| **Formula 4** | **% by weight** |
|---|---|
| Dimethicone 10 cst | 22.50 |
| Dimethicone and dimethicone crosspolymer | 10.00 |
| Polymethylsilsesquioxane | 67.00 |
| Active ingredients | 0.50 |

### EXAMPLE 5

| **Formula 5** | **% by weight** |
|---|---|
| Dimethicone 350 cst | 30.00 |
| Dimethicone and dimethicone crosspolymer | 10.00 |
| Polymethylsilsesquioxane | 60.00 |

### EXAMPLE 6

| **Formula 6** | **% by weight** |
|---|---|
| Dimethicone 10 cst | 20.00 |
| Dimethicone and dimethicone crosspolymer | 9.50 |
| Polymethylsilsesquioxane | 70.00 |
| Pigments and pearls | 0.50 |

The product is obtained by extruding a paste created by mixing the above-described ingredients, followed by cutting and pressing into containers.

Main features of this product are the absolute transparency thereof and the change of texture during its application, because once taken by means of a specific applicator it turns from rubbery into powder.

These features originate from the association of polymethylsilsesquioxane with silicone oils, such as dimethicone, with a viscosity in the range from 10 to 350 cst, and with silicone resins, such as for example dimethicone and dimethicone crosspolymer.

The product is obtained by premixing the liquid or semi-liquid or pasty ingredients in a specific mixer equipped with planetary blades along with possible pigments and active ingredients, such as vitamins, solar filters, antioxidants.

The powder phase consisting of polymethylsilsesquioxane is then added and subjected to further mixing until an extrudable paste is formed.

The product thus obtained is in the form of a white paste which becomes transparent when it is extruded.

Once it has been cut into the desired shapes, the extruded product is put into specific containers and pressed.

## Claims

1. Cosmetic product with smoothing effect **characterized by** comprising:
• from 60 to 70 % by weight of polymethylsilsesquioxane;
• from 30 to 40 % by weight of silicone oils and silicone resins.

2. Cosmetic product according to claim 1, **characterized in that** the silicone oils and resins consist of:
• from 20 to 30 % by weight of dimethicone;
• from 5 to 10 % by weight of dimethicone and dimethicone crosspolymer.

3. Cosmetic product according to claim 2, **characterized in that** dimethicone with ist viscosity from 10 to 350 cst is used.

4. Cosmetic product according to any preceding claim, **characterized by** comprising active ingredients, solar filters, pigments, pearls.

5. Process for obtaining a product according to any preceding claim, **characterized by** comprising a mixing step, an extrusion step, a cutting step and a final step of pressing into suitable containers.

## Patentansprüche

1. Kosmetisches Produkt mit glättendem Effekt, **dadurch gekennzeichnet, dass** es umfasst:
- 60 bis 70 Gewichts-% Polymethylsilsesquioxan;
- 30 bis 40 Gewichts-% Silikonöle und Silikonharze.

2. Kosmetisches Produkt gemäß Anspruch 1, **dadurch gekennzeichnet, dass** die Silikonöle und Silikonharze bestehen aus:
- 20 bis 30 Gewichts-% Dimeticon;
- 5 bis 10 Gewichts-% Dimeticon und Dimeticon-Crosspolymer

3. Kosmetisches Produkt gemäß Anspruch 2, **dadurch gekennzeichnet, dass** Dimeticon mit einer Viskosität von 10 bis 350 cst verwendet wird.

4. Kosmetisches Produkt gemäß einem der vorherigen Ansprüche, **dadurch gekennzeichnet, dass** es aktive Inhaltsstoffe, Sonnenfilter, Pigmente, Perlen aufweist.

5. Verfahren zur Herstellung eines Produkts gemäß einem der vorherigen Ansprüche, **dadurch gekennzeichnet, dass** es einen Mischungsschritt, einen Extrusionsschritt, einen Schneideschritt und einen finalen Schritt des Verpressens in geeignete Behälter umfasst.

## Revendications

1. Produit cosmétique ayant un effet lisseur **caractérisé par le fait qu'**il comprend :
• de 60 à 70 % en poids de polyméthylsilsesquioxane ;
• de 30 à 40 % en poids d'huiles de silicone et de résines de silicone.

2. Produit cosmétique selon la revendication 1, **caractérisé en ce que** les huiles et les résines de silicone sont constituées de :
• de 20 à 30 % en poids de diméthicone ;
• de 5 à 10 % en poids de diméthicone et de polymère réticulé de diméthicone.

3. Produit cosmétique selon la revendication 2, **caractérisé en ce qu'**une diméthicone ayant une viscosité de 10 à 350 est est utilisée.

4. Produit cosmétique selon l'une quelconque des revendications précédentes, **caractérisé par le fait qu'**il comprend des substances actives, des filtres solaires, des pigments, des perles.

5. Procédé permettant d'obtenir un produit selon l'une quelconque des revendications précédentes, **caractérisé par le fait qu'**il comprend une étape de mélange, une étape d'extrusion, une étape de découpe et une étape finale de compression dans des récipients adéquats.
